# EUROPÄISCHE PATENTSCHRIFT

(11) **EP 1 371 338 B1**
(45) Veröffentlichungstag und Bekanntmachung des Hinweises auf die Patenterteilung: **08.03.2006**
(21) Anmeldenummer: 03013095.9
(22) Anmeldetag: 11.06.2003
(51) Int. Cl.: A61B 17/70

(54) **Knochenschraube**
Bone screw
Vis pédiculaire

(30) Priorität: 11.06.2002 DE 20209025 U
(43) Veröffentlichungstag der Anmeldung: 17.12.2003
(73) Patentinhaber: Signus Medizintechnik GmbH, 63755 Alzenau (DE)
(72) Erfinder: Dierks, Michael, 42929 Wermelskirchen (DE); Kretschmer, Peter, Dipl.-Ing., 63486 Bruchköbel (DE)
(74) Vertreter: Gudat, Axel

(56) Entgegenhaltungen:
- WO-A-98/49961
- DE-A- 19 950 075
- FR-A- 2 786 088
- US-A- 5 261 909
- US-A- 5 344 422
- US-A- 5 876 403
- US-B1- 6 478 798

## Beschreibung

Die Erfindung betrifft eine Knochenschraube mit einem in einem Knochen, insbesondere in einen Wirbel, befestigbaren Schraubenschaft und einem Schraubenkopf, an dem eine Befestigungseinrichtung mit einer Aufnahme zur Halterung eines Verbindungsstabes befestigbar ist, wobei die Befestigungseinrichtung in der Neigung gegen den Schraubenschaft verschwenkbar ist, wobei der Schraubenkopf eine Ausnehmung aufweist, in der ein gegenüber dem Schraubenschaft verschwenkbares und lagefixierbares Schwenkelement zur Befestigung einer Befestigungseinrichtung zur Festlegung eines Verbindungsstabes und/oder eines Befestigungsbereich eines Verbindungsstabes und/oder eines gegebenenfalls vorgesehenen Adapters angeordnet ist, wobei das Schwenkelement mit einem Befestigungsbereich versehen ist, an dem der Adapter und/oder die Befestigungseinrichtung zur Fixierung eines Verbindungsstabes festgelegt ist und wobei ein Befestigungsmittel vorgesehen ist, mittels dessen die Befestigungseinrichtung betätigbar und gleichzeitig die Befestigungseinrichtung oder der Adapter unter Lagefixierung des Schwenkelementes gegen den Schraubenkopf fixierbar ist.

Derartige Knochenschrauben oder diese umfassende Vorrichtungen sind vielfältig bekannt. So ist es beispielsweise bekannt, Pedikelschrauben mit kugelartigem Schraubenkopf zu verwenden, auf welchem eine ein- oder zweiteilige Klammer aufgesetzt ist, mittels derer ein Verbindungsstab festklemmbar und in verschiedenen Verschwenkstellungen gegenüber dem Schraubenschaft festlegbar ist. Der Verschwenkbereich des Verbindungsstabes gegenüber dem Schraubenschaft ist hierbei jedoch vergleichsweise eingeschränkt. Ferner sind gattungsgemäße Knochenschrauben bekannt, bei welchen eine Verschwenkung der Klemmeinrichtung gegenüber dem Schraubenschaft über einen größeren Winkelbereich möglich ist, bei welcher jedoch bei der Anwendung der Knochenschraube oder der dieser zugeordneten Vorrichtung sich die Klemmeinrichtung unbeabsichtigt relativ weit von ihrer Sollposition entfernen kann oder durch unbeabsichtigte Lösung des Mittels zur Lagefixierung der Klemmeinrichtung gegenüber dem Schraubenschaft eine unbeabsichtigte Demontage der Klemmeinrichtung erfolgen kann.

Die WO 98/49961 (Basis für den Oberbegriff des Anspruchs 1.) beschreibt eine Knochenschraube mit einem Bolzen, welcher ein kugelförmiges erstes Ende und ein zweites Gewindeende zur Aufnahme einer Verschlussmutter aufweist, wobei ferner an dem hülsenartigen Schraubenkopf eine Befestigungskappe zur Verbindung des Bolzens mit dem Schraubenkopf vorgesehen ist. Die Befestigungskappe ist in den hülsenförmigen Schraubenkopf eingeschraubt und weist eine abgerundete Innenoberfläche sowie eine zentral angeordnete Öffnung auf, in welcher der Bolzen angeordnet und in verschiedenen Winkelstellungen festlegbar ist.

Die US 5,876,403 beschreibt eine Knochenschraube mit einem gabelförmigen Schwenkelement, welches um einen in einer seitlichen Öffnung des Schraubenkopfes positionierten Stift verschwenkbar ist. Das Schwenkelement wird in seiner jeweiligen Schwenkstellung durch eine separate Madenschraube festgelegt. Zur Festlegung eines Verbindungsstabes an einer entsprechenden Befestigungseinrichtung sind zwei Muttern auf gegenüberliegenden Seiten der Befestigungseinrichtung vorgesehen, um den Verbindungsstab unter Kraftbeaufschlagung festzulegen.

Der Erfindung liegt die Aufgabe zugrunde, eine gattungsgemäße Knochenschraube bereitzustellen, die eine Verschwenkung einer Befestigungseinrichtung gegenüber dem Schraubenschaft um einen möglichst großen Winkelbereich ermöglicht und die einfach und sicher handhabbar ist.

Diese Aufgabe wird erfindungsgemäß durch eine Knochenschraube nach Anspruch 1 gelöst. Der Schraubenkopf weist hierbei eine Ausnehmung auf, in der ein gegenüber dem Schraubenschaft verschwenkbares und lagefixierbares Schwenkelement angeordnet ist, an dem eine Befestigungseinrichtung befestigt ist. Dadurch, dass das Schwenkelement in einer Ausnehmung des Schraubenkopfes gehaltert ist, kann durch die Bemessung der Weite der Ausnehmung in Verschwenkrichtung auch der Verschwenkwinkel begrenzt werden, so dass sich das Schwenkelement nicht über ein unerwünschtes Ausmaß aus seiner Sollposition bewegen kann. Ferner ist hierdurch eine besonders kompakte und stabile Bauform der Knochenschraube und der Gesamtanordnung mit zugeordnetem Adapter und Befestigungseinrichtung im Bereich des Adapters und des Befestigungsbereich für den Verbindungsstab möglich. Die Schwenkachse kann bei geeigneter Ausrichtung des Verbindungsstabes parallel zur Längsachse desselben verlaufen, wobei bei der Verdrehbewegung die Drehachse und die Verbindungsstablängsachse stets einen Winkel zueinender einschließen können, vorzugsweise einen Winkel von 90 Grad. Ferner kann das Schwenkelement im entarretierten Zustand, in welchem eine Verschwenkung über den gesamten Verschwenkwinkel möglich ist, sicher und unverlierbar an der Schraube festgelegt sein. Hierzu kann z.B. das Schwenkelement mit einer Verbreiterung versehen sein, die z.B. an dem Kopfende desselben angeordnet sein kann, oder es können die weiter unten beschriebenen Sicherungsmittel oder andere geeignete Mittel vorgesehen sein.

Die Erfindung wird durch die Merkmale der Unteransprüche weitergebildet.

Die Erfindung umfasst eine Knochenschraube mit an dieser bestimmungsgemäß angeordnetem Schwenkelement, Adapter und/oder Befestigungseinrichtung, die zusammen zumindest teilweise oder vollständig eine Vorrichtung zur Halterung eines Verbindungsstabes eines spinalen Fixierelementes bilden.

Die Befestigungseinrichtung zur Festlegung des Verbindungsstabes, die an der erfindungsgemäßen Vorrichtung anordenbar oder von dieser umfasst ist, kann insbesondere als Klemmeinrichtung ausgeführt sein, ohne hierauf beschränkt zu sein. Hierzu kann ein Klemmmittel, beispielsweise eine Klemmschraube, vorgesehen sein, mittels derer der Verbindungsstab an der erfindungsgemä-ßen Vorrichtung befestigbar ist. Das Befestigungsmittel kann mittelbar auf den Verbindungsstab oder auf eine Verlängerung desselben wirken, beispielsweise vermittels einer Klemmschelle. Das Befestigungsmittel, insbesondere in Form eines Klemmmittels, kann auch unmittelbar an einem Befestigungsbereich des Verbindungsstabes angreifen, der beispielsweise in Form einer Öse ausgeführt sein kann, durch die das Schwenkelement durchführbar ist, so dass der Befestigungsbereich des Verbindungsstabes an die erfindungsgemäße Vorrichtung angedrückt werden kann. Die Öse kann hierbei seitlich offen oder geschlossen ausgeführt sein und ggf. ein- oder beidseitig mit einer Verzahnung versehen sein. Die Befestigungseinrichtung kann auch in anderer geeigneter Weise vorzugsweise form- und/oder kraftschlüssig wirken.

Das Schwenkelement kann in der Ausnehmung überwiegend oder ausschließlich monoaxial in einer Raumrichtung um eine definierte Achse verschwenkbar sein und eine seitliche Führung, vorzugsweise eine beidseitige Führung aufweisen, so dass das Schwenkelement mit nur geringem oder praktisch ohne seitliches Spiel und/oder mit nur geringer oder im wesentlichen ohne seitliche Verkippungsbewegung verschwenkbar ist. Gegebenenfalls kann das Schwenkelement jedoch auch in zwei oder mehreren einen Winkel zueinander einschließenden Richtungen verschwenkbar sein, beispielsweise im Bereich einer Kugelkalotte. Die seitliche Begrenzung der Verschwenkbarkeit und/oder Verschiebbarkeit des Schwenkelementes, die insbesondere jeweils spielfrei oder mit geringem Spiel erfolgen kann, kann durch die seitlichen Ränder der Ausnehmung oder durch andere geeignete Mittel erfolgen.

Der Verschwenkwinkel kann ≥ ± 5 Grad, vorzugsweise ≥ ± 25 Grad, beispielsweise ≥ ± 45 Grad betragen, ohne hierauf beschränkt zu sein. Die neutrale Position der Schwenkachse des Schwenkelementes, d.h. die Stellung des Schwenkelementes bei maximaler Verschwenkung zur Schraubenschaftlängsachse hin, kann in einem Winkel von 0° bis 45° zur Längsachse des Schraubenschaftes liegen. Bei einem Winkel von 0° bildet das Schwenkelement somit die axiale Verlängerung des Schraubenschaftes.

Die Schwenkachse des Schwenkelementes schließt vorzugsweise einen Winkel 90 Grad zu der Längsachse des Schraubenschaftes ein.

Die Ausnehmung des Schraubenkopfes zur Aufnahme des Schwenkelementes kann taschenförmig ausgebildet sein, insbesondere in Art einer Sacköffnung, so dass durch die Ränder der Ausnehmung der Verschwenkwinkel und/oder eine ein- oder beidseitige Bewegung des Schwenkelementes begrenzt wird. Das Schwenkelement kann zur Festlegung an dem Schraubenkopf von vorne in die Sacköffnung eingeführt werden. Gegebenenfalls kann das Schwenkelement an Haltemitteln des Schraubenkopfes angreifen, wozu das Schwenkelement derart verdrehbar sein kann, dass es einen oder mehrere an der Schraube, insbesondere im Bereich der Ausnehmung, angeordnete Vorsprünge hintergreift. Gegebenenfalls kann die Ausnehmung zur Aufnahme des Schwenkelementes auch als Durchtrittsöffnung mit einer rückseitigen Öffnung an dem Schraubenkopf oder als seitlich offene Aufnahme ausgeführt sein, so dass das Schwenkelement in einer Richtung in den Schraubenkopf eingeführt werden kann, die von der Richtung verschieden ist, in der das Schwenkelement von dem Schraubenkopf nach außen hervorsteht, insbesondere der entgegengesetzten Richtung. Das Schwenkelement kann hierbei beispielsweise im wesentlichen stiftförmig oder auch bügelförmig ausgeführt sein. Das Schwenkelement kann auch in zwei benachbarte Ausnehmungen des Schraubenkopfes eingreifen, die sich aufeinander zugewandt oder in unterschiedliche Richtungen, z.B. entgegengesetzt, öffnen. Die Ausnehmungen können z.B. an gegenüberliegenden Seiten des Schraubenkopfes vorgesehen sein.

Vorzugsweise ist die Aufnahme für das Schwenkelement auf Höhe des Schraubenschaftes angeordnet.

Vorzugsweise ist eine seitliche, vorzugsweise beidseitige, Führung für das Schwenkelement vorgesehen, die im entarretierten Zustand des Schwenkelementes auf dieses wirkt, so dass dieses gegen seitliche Verschiebung und/oder Verschwenkung spielfrei oder mit geringem Spiel gesichert ist.

Ferner ist ein separates Halteelement vorgesehen, welches das entarretierte und/oder arretierte Schwenkelement gegen Entfernung, insbesondere durch Längsverschiebung, aus der Aufnahme sichert bzw. in dieser hält. Dieses separate Halteelement ist vorzugsweise zusätzlich zu dem Mittel zur Lagefixierung des Schwenkelementes gegenüber dem Schraubenschaft vorgesehen und unabhängig von diesem betätigbar, so dass auch bei vollständiger Entfernung des Elementes, welches das Schwenkelement in einer gewählten Schwenkstellung lagefixiert das Schwenkelement an dem Schraubenkopf sicher gehaltert ist. Das Fixierungselement kann das Befestigungsmittel zur Festlegung des Verbindungsstabes darstellen, beispielsweise die weiter unten beschriebene Klemmmutter, ohne hierauf beschränkt zu sein. Hierdurch wird die Handhabbarkeit der erfindungsgemäßen Vorrichtung wesentlich verbessert, da auch bei vollständiger Entarretierung des Schwenkelementes ein unbeabsichtigtes Lösen desselben von der Schraube verhindert wird. Das Sicherungsmittel kann das Schwenkelement durchgreifen oder in dieses eingreifen, es kann das Schwenkelement auch lediglich hintergreifen und so beispielsweise gegen Herausfallen in der Einschubrichtung in den Schraubenkopf sichern.

Das separate Halteelement kann als Haltestift oder Haltebolzen ausgeführt sein, welcher in einer Öffnung des Schraubenkopfes eingeführt ist, die in der Ausnehmung zur Aufnahme des Schwenkelementes mündet, so dass das separate Halteelement an dem Schwenkelement angreifen kann. Vorzugsweise ist die Ausnehmung zur Aufnahme des separaten Halteelementes als Durchgangsöffnung ausgeführt, die beidseitig, vorzugsweise auf gegenüberliegenden Stirnseiten, des Schraubenkopfes ausmündet. Die Stirnseiten können die Stirnflächen eines als Querstab ausgebildeten Schraubenkopfes darstellen. Das separate Halteelement schließt hierbei vorzugsweise an beiden Enden bündig mit den Stirnseiten des Schraubenkopfes ab und verschließt vorzugsweise die Einführöffnung vollständig. Durch das separate Halteelement kann zugleich die Verschwenkachse des Schwenkelementes definiert werden.

Das separate Halteelement, das insbesondere als Haltestift ausgeführt sein kann, kann an dem Schraubenkopf verschiebungs- und/oder verdrehsicher festgelegt werden, wozu geeignete Kraftschluss- und/oder Formschlussmittel oder geeignete andere Mittel vorgesehen sein können, beispielsweise in Form eines Schraubgewindes. Anstelle eines Haltestiftes kann auch ein anderes geeignetes Haltemittel vorgesehen sein.

Das separate Halteelement haltert das Schwenkelement gegen Längsverschiebung, vorzugsweise in jeder der bestimmungsgemäßen Verschwenkstellungen, wobei eine Längsverschiebung vollständig oder teilweise durch das separate Halteelement unterbunden wird. Hierzu kann das Schwenkelement einen hakenförmigen Vorsprung, vorzugsweise eine Öse, aufweisen, welcher das separate Halteelement zumindest teilweise hintergreift bzw. in welche das separate Halteelement einführbar ist. Der Innendurchmesser der Öse entspricht hierbei vorzugsweise dem Durchmesser des eingreifenden Haltestiftes. Vorzugsweise entspricht der Durchmesser des separaten Halteelementes an seinem Angriffsbereich mit dem Schwenkelement dem Durchmesser des separate Halteelementes an einem oder an beiden Endbereichen desselben, beispielsweise in Form eines Stiftes, der über seine Länge einen konstanten Durchmesser aufweist.

Das von dem Schraubenkopf nach außen vorstehende Ende des Schwenkelementes kann einen Haltebereich zur Festlegung einer Befestigungseinrichtung und/oder eines zwischen Befestigungseinrichtung und Schraubenkopf anordenbaren Adapters aufweisen, wobei die Befestigungseinrichtung jeweils als Klemmeinrichtung ausgeführt sein kann. Der Haltebereich kann hierzu mit einem Gewindeabschnitt versehen sein.

Vorzugsweise ist der Schraubenkopf als sich ein- oder beidseitig von dem Schraubenschaft erstreckender Fortsatz, der sich senkrecht oder in einem anderen Winkel zu dem Schaft erstrecken kann, oder als Kugelkopf ausgeführt, ohne hierauf beschränkt zu sein. Der Schraubenkopf bildet vorzugsweise den axial am weitesten von dem Schraubenschaft entfernten Teil der Pedikelschraube. Der Schraubenkopf kann, unabhängig von seiner sonstigen Ausführung enteilig oder lösbar an dem Schaft befestigt sein.

Besonders bevorzugt ist zumindest der Teilbereich des Schraubenkopfes an dem der Adapter und/oder die Befestigungseinrichtung festlegbar ist oder der gesamte Schraubenkopf in Form eines Rotationskörpers oder eines vorzugsweise nach außen gekrümmten Teilabschnittes desselben ausgeführt. Der Rotationskörper weist vorzugsweise eine Hauptachse mit einer größeren Länge als Achsen in anderen Richtungen auf, wobei das Schwenkelement um diese Hauptachse verschwenkbar ist. Der Rotationskörper kann beispielsweise als Zylinderabschnittes ausgeführt, so dass die Umfangsfläche des Schraubenkopfes eine Zylinderfläche oder Teilzylinderfläche darstellt, wobei die Verschwenkachse des Schwenkelementes parallel bzw. koaxial zu der Zylinderachse angeordnet ist. Es ist hierzu ausreichend, wenn lediglich ein- oder beidseitig und/oder ober- und/oder unterseitig der Aufnahme für das Schwenkelement angeordnete Bereiche des Schraubenkopfes in Form von Zylinderflächen vorgesehen sind, so dass ein an dem Schwenkelement angeordneter Adapter und/oder Klemmeinrichtung eine Schwenkbewegung um die Schraubenkopfachse unter währender Anlage einer oder mehreren Zylinderflächen durchführen können. Die Ausnehmung zur Aufnahme des Schwenkelementes ist hierbei vorzugsweise in einer Zylinder- oder Teilzylinderfläche des Schraubenkopfes eingebracht. Vorzugsweise ist zumindest der Bereich des Schraubenkopfes, der bei maximalem Verschwenkwinkel von dem Adapter und/oder der Schwenkeinrichtung übergriffen wird, als Zylinderfläche ausgeführt. Konstruktiv besonders einfach ist es, wenn der Schraubenkopf insgesamt als zylinderförmiger Stab ausgebildet ist. Die Stirnseiten des Schraubenkopfes können hierbei eben und parallel zueinander oder in anderer geeigneter Weise ausgeführt sein. Entsprechendes gilt, wenn der Fortsatz die Gestalt eines anderen Rotationskörpers ausweist, z.B. eines tonnenförmigen Körpers, eines Rotationsellipsoids oder dergleichen.

Gegebenfalls kann der Schraubenkopf auch als Teil einer Kugeloberfläche ausgeführt sein, um eine Verschwenkung des Schwenkelementes in einer oder in mehreren Raumrichtungen zu ermöglichen, wobei die Ausnehmung zur Aufnahme des Schwenkelementes vorzugsweise in dem zweidimensionalen Kugeloberflächenabschnitt eingebracht ist.

Nach einer bevorzugten Ausführungsform ist ein an dem Schwenkelement festlegbarer Adapter vorgesehen, der eine linienförmig oder vorzugsweise flächig an dem Schraubenkopf anlegbare Anlagefläche aufweist, die vorzugsweise als konvex gekrümmte Fläche ausgeführt ist, sowie eine weitere Anlagefläche für eine Befestigungseinrichtung, insbesondere eine Klemmeinrichtung, so dass die Befestigungseinrichtung bei Bedarf an dem Adapter befestigbar ist. Die konkave, nach innen eingebauchte Fläche kann eine Teilfläche eines Rotationskörpers darstellen, der vorzugsweise eine Hauptachse mit einer größeren Länge als Achsen in anderen Richtungen hat, z.B. eine Zylinderteilfläche oder Teilfläche einer Tonne, Rotationsellipsoid oder dergleichen. Die dem Schraubenkopf zugewandte Einbuchtung des Adapters kann teilzylinderförmig ausgeführt sein. Unabhängig hiervon oder in Kombination hiermit kann die der Anlagefläche des Adapters an dem Schraubenkopf gegenüberliegende Adapterfläche eben ausgeführt sein. Die Ausführungen zu der Gestalt der Adapterfläche können entsprechend für eine Anlagefläche eines Befestigungselementes, z.B. eines Klemmelementes wie eine Bügelschelle, an dem Schraubenkopf gelten.

Die der Befestigungseinrichtung zugeordnete Anlagefläche des Adapters, an die die Befestigungseinrichtung mit einer Anlage ablegbar ist, kann im wesentlichen eben, sphärisch konvex, sphärisch konkav oder konisch (männlich oder weiblich konisch) ausgeführt sein. Die Hauptachse der konvexen, konkaven oder konische Sphäre ist vorzugsweise parallel zur Längsachse des Schwenkelementes angeordnet. Die korrespondierende Anlagefläche des Befestigungselementes kann die gleiche oder eine andere Raumkrümmung haben wie die Anlagefläche des Adapters.

Vorzugsweise ist die Befestigungseinrichtung, insbesondere eine Klemmeinrichtung, gegenüber dem Adapter um eine Achse verdrehbar ausgeführt, wobei die Verdrehachse vorzugsweise koaxial zu der Längsachse des Schwenkelementes angeordnet ist. Gegebenenfalls können die Drehachse der Befestigungseinrichtung und die Längsachse des Schwenkelementes auch einen Winkel zueinander einschließen oder versetzt zueinander sein.

Vorzugsweise weist der Adapter eine Durchtrittsöffnung zur Durchführung des Schwenkelementes und die Befestigungseinrichtung eine Aufnahme für den Befestigungsbereich des Schwenkelementes auf, die auch als Durchtrittsöffnung ausgebildet sein kann. Der Adapter und die Befestigungseinrichtung können hierdurch auf das Schwenkelement aufgeschoben und gemeinsam gegenüber dem Schraubenschaft verschwenkt werden. Der Adapter ermöglicht hierbei eine großflächige Anlage sowohl an der gekrümmten Schraubenkopfoberfläche als auch an der Befestigungseinrichtung.

Gegebenenfalls kann die Befestigungseinrichtung auch eine bogenförmige Fläche, insbesondere in Form eines Umfangsabschnittes eines Rotationskörpers wie eines Zylinders oder Rotationsellipsoids, aufweisen, die an die Schraubenkopfoberfläche anlegbar ist, so dass die Befestigungseinrichtung auch unmittelbar an dem Schraubenkopf festlegbar ist.

Vorzugsweise sind zumindest eine oder beide der jeweils einander zugewandten Anlageflächen von Schraubenkopf und Adapter und/oder Adapter und Befestigungseinrichtung aufgeraut, insbesondere verzahnt, wodurch die Verdrehsicherheit der genannten Bauteile zueinander erhöht wird. Insbesondere können beide Anlageflächen des Adapters an den Schraubenkopf und das Befestigungselement aufgeraut, insbesondere verzahnt, sein. Dies ist insbesondere dann zweckdienlich, wenn die Befestigungseinrichtung unmittelbar an dem Schraubenkopf festgelegt wird und nur vergleichsweise kleine Anlageflächen miteinander zum Angriff kommen, beispielsweise dann, wenn die Anlageflächen unterschiedliche Krümmungen im Raum aufweisen. Eine oder beide der Anlageflächen der oben genannten Flächenpaarungen können jedoch auch im wesentlichen glatt ausgeführt sein.

Insbesondere kann die Anlagefläche des Adapters für den Schraubenkopf und/oder das Befestigungselement aufgeraut, insbesondere verzahnt oder radialverzahnt, oder auch glatt ausgeführt sein. Unabhängig hiervon oder gleichzeitig hiermit können auch die dem Adapter zugeordneten Anlageflächen der Befestigungseinrichtung und/oder des Schraubenkopfes aufgeraut, insbesondere verzahnt, oder auch glatt ausgeführt sein.

Insbesondere kann die der Befestigungseinrichtung zugewandte Anlagefläche des Adapters eine Radialverzahnung aufweisen, insbesondere bei planer, sphärisch konkaver oder sphärisch konvexer Formung der Adapteranlagefläche. Die Anlagefläche kann auch rau ausgeführt sein, insbesondere bei planer, sphärisch konkaver oder sphärisch konvexer Formung. Die Anlagefläche auch glatt ausgeführt sein, insbesondere bei planer, sphärisch konkaver oder sphärisch konvexer Formung. Die Anlagefläche kann auch bei männlich konischer oder weiblich konischer Formung eine Verzahnung, insbesondere Radialverzahnung, aufweisen oder glatt oder rau ausgeführt sein. Die korrespondierende Anlagefläche der Befestigungseinrichtung kann jeweils eine Verzahnung, insbesondere Radialverzahnung, aufweisen oder glatt oder rau ausgeführt sein, insbesondere bei jeweils korrespondierender Formung zu der Adapteranlagefläche, die jeweils eine linienförmige oder flächige Anlage aneinander erlauben.

Vorzugsweise weisen der Adapter und/oder die Befestigungseinrichtung eine Erstreckung in Verschwenkrichtung auf, so dass die Aufnahme für das Schwenkelement in jeder der möglichen Verschwenkstellungen vollständig von dem Adapter und/oder der Befestigungseinrichtung abgedeckt ist.

Der Adapter und/oder die Befestigungseinrichtung können sich über die gesamte Breite des Schraubenkopfes erstrecken, ohne hierauf beschränkt zu sein.

Erfindungsgemäß ist ferner die Befestigungseinrichtung als Klemmeinrichtung ausgeführt, wobei die Verspannung der Klemmeinrichtung zur Festlegung des Verbindungsstabes durch das gleiche Klemmmittel erfolgt wie die Lagefixierung von Adapter und/oder Klemmeinrichtung um die Verschenkachse. Hierzu kann beispielsweise ein Klemmelement, z.B. eine Spannschraube, vorgesehen sein, welche an einem Gewindeabschnitt des Schwenkelementes festlegbar ist, so dass durch Betätigung der Spannschraube die Klemmeinrichtung gespannt und gleichzeitig gegenüber den Adapter kraftbeaufschlagt wird, wobei der Adapter gegenüber dem Schraubenkopf kraftbeaufschlagt wird und so das Schwenkelement an dem Schraubenkopf lagefixiert wird. Gegebenenfalls können hierzu auch jeweils separate Feststellmittel vorgesehen sein.

Bei der erfindungsgemäßen Vorrichtung kann der Schraubenkopf exzentrisch zu dem Schraubenschaft angeordnet sein, so dass die Verschwenkachse seitlich versetzt zu der Schraubenschaftlängsachse angeordnet ist. Die Verschwenkachse kann innerhalb des Durchmessers des Schraubenschaftes oder außerhalb desselben angeordnet sein. Der seitliche Versatz kann bis ¼ bis ½ oder bis zum ganzen Radius oder Durchmesser des Schraubenschaftes auf Höhe des Schraubenkopfes betragen, ohne hierauf beschränkt zu sein. Der seitliche Versatz kann weniger oder mehr als 1 mm, im Bereich von ca. 1 mm bis ca. 10 mm oder bis ca. 20 mm, vorzugsweise im Bereich von ca. 3 bis ca. 5 mm betragen, ohne hierauf beschränkt zu sein. Vorzugsweise ist die Exzentrizität derart gewählt, dass bei an dem Wirbel anliegender und zwischen Dorn- und Querfortsatz angeordneter Befestigungseinrichtung zwischen Außenseite der Befestigungseinrichtung und Dornfortsatz ein derartiger Abstand verbleibt, dass sich die Befestigungseinrichtung an jeder Etage der Wirbelsäule in jedem Fall ohne Restriktion und somit ohne implantationsbedingte Traumatisierung der Dornfortsätze frei montieren lässt.

Die Erfindung wird nachfolgend beispielhaft beschrieben und anhand der Figuren beispielhaft erläutert. Es zeigen:
- Fig. 1: Ein erstes Ausführungsbeispiel einer erfindungsgemäßen Knochenschraube mit zugeordneten Bauteilen in Explosionsdarstellung,
- Fig. 2: ein weiteres Ausführungsbeispiel einer erfindungsgemäßen Knochenschraube,
- Fig. 3: ein weiteres Ausführungsbeispiel einer Klemmeinrichtung für eine erfindungsgemäße Knochenschraube.

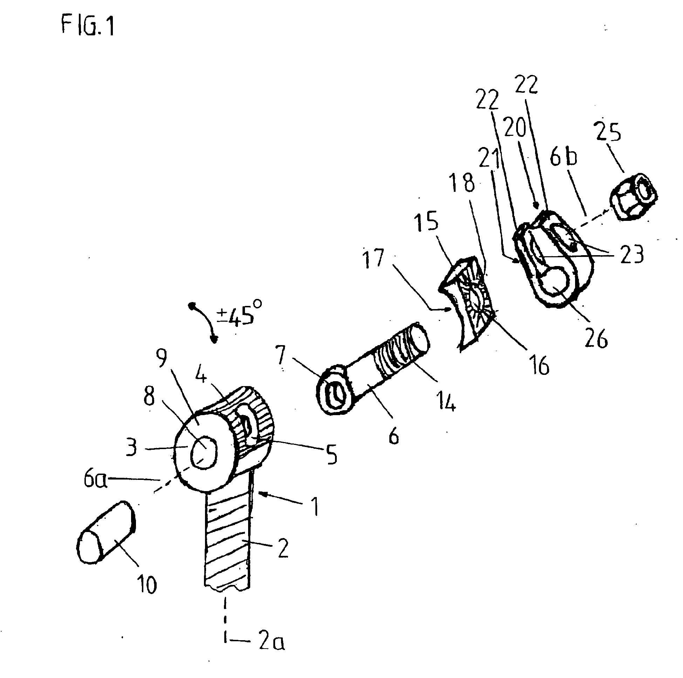

Fig. 1 zeigt eine erfindungsgemäße Knochenschraube 1 als Bestandteil einer Vorrichtung zur Halterung eines Verbindungsstabes eines spinalen Fixiermittels, wobei die Knochenschraube einen Schaft 2 und einen Schraubenkopf 3 aufweist. Der Schraubenkopf ist in Art eines zylindrischen Querstabes ausgeführt, welcher senkrecht zu der Längsachse 2a des Schaftes angeordnet ist und beidseitig von dem Schaft 2 vorsteht. In der zylindrischen Umfangsfläche 4 des Schraubenkopfes ist eine Ausnehmung 5 in Art eines Langloches ausgebildet, in welche das Ende des stabförmigen Schwenkelementes 6 einführbar ist. Das Schwenkelement ist in der Ausnehmung um einen Winkelbetrag von ca. ± 45 Grad verschwenkbar und schließt in seiner dem Schaft 2 zugewandten maximalen Schwenkstellung mit diesem einen Winkel von ca. 90 Grad ein. Die neutrale Position der Achse des Schwenkelementes, in der dieses maximal zur Schraubenschaftlängsachse verschwenkt ist, steht hier in einem Winkel von ca. 20° zur Längsachse des Schraubenschaftes, d.h. schräg nach oben, wie in Fig. 1 verdeutlicht. Die Breite der Ausnehmung 5 ist hierbei derart bemessen, dass das Schwenkelement 6 praktisch spielfrei an beiden seitlichen Begrenzungen der Ausnehmung 5 anliegt oder geringfügig von diesen absteht.

Zur verschiebungssicheren und unverlierbaren Halterung des Schwenkelementes 6 ist dieses an dem in der Ausnehmung 5 anordenbaren Ende mit einer Öse 7 versehen. Ferner ist der Schraubenkopf 3 mit einer sich in Längsrichtung erstreckenden Durchgangsbohrung 8 versehen, welche an beiden Stirnseiten 9 des Kopfes 3 mündet und die Ausnehmung 5 durchschneidet. Die Öffnung der Öse 7 ist hierbei fluchtend zu der Durchgangsbohrung 8 anordenbar, so dass ein Verschlussbolzen 10 in die Durchgangsbohrung 8 einführbar und durch die Öse 7 durchführbar ist, wobei der Verschlussbolzen bündig an beiden Stirnseiten 9 endet oder von einer oder beiden Stirnseiten nach außen vorstehen oder zurückversetzt sein kann. Der Verschlussbolzen 10 kann kraftschlüssig, durch ein Gewinde oder durch andere geeignete Mittel verschiebungssicher an dem Schraubkopf 3 festgelegt werden. Der Außendurchmesser des Bolzens 10 entspricht im Verbindungsbereich dem Innendurchmesser der Öse 7, so dass das Schwenkelement in Längsrichtung vorzugsweise im wesentlichen spielfrei oder mit geringem Spiel an dem Schraubenkopf festgelegt ist. Der Bolzen 10 kann von einer oder von beiden Stirnseiten 9 des Schraubenkopfes 3 her in die korrespondierende Öffnung, die als Sackbohrung oder als Durchgangsbohrung ausgeführt sein kann, eingeschoben werden, wobei der Bolzen 10 die Einführöffnung vollständig verschließt.

Die erfindungsgemäße Vorrichtung weist ferner einen Adapter 15 auf, der eine Durchgangsbohrung 16 zur Durchführung des Schwenkelementes 6 aufweist und an seiner dem Schraubenkopf 3 gegenüberliegenden Fläche 17 eine zylinderförmige Einbuchtung aufweist, so dass der Adapter vollflächig an die zylinderförmige Umfangsfläche 4 des Schraubenkopfes 3 anlegbar und in einer Verschwenkbewegung auf dieser Fläche aufliegend entlanggeführt werden kann. Die gegenüberliegende Fläche 18 des Adapters ist eben ausgeführt und kann an die ebene Unterseite 21 des Befestigungselementes für einen Verbindungsstab, das nach dem Ausführungsbeispiel als Klemme 20 ausgeführt ist, angelegt werden. Die Klemme ist hier als Bügelschelle ausgeführt, sie kann aber auch auf andere geeignete Weise ausgeführt sein, um den Verbindungsstab festzulegen. Die Klemme kann hierbei um die Richtung 6b verdreht werden. Die beiden mit Durchgangsbohrungen 23 versehenen Schenkel 22 der Klemme können auf das Schwenkelement 6 aufgesetzt werden, welches an seinem Ende mit einem Gewindeabschnitt 14 versehen ist. Die Klemmmutter 25 kann auf den Gewindeabschnitt 14 aufgeschraubt werden, wodurch die beiden Schenkel 22 zusammengepresst und ein in der Aufnahme 26 angeordneter Verbindungsstab festklemmbar ist. Gleichzeitig wird mittels der Mutter 25 die Klemme gegen den Adapter und dieser gegen die zylindrische Umfangsfläche 4 des Schraubenkopfes kraftbeaufschlagt, wodurch gleichzeitig das Schwenkelement und damit auch der Adapter und die Klemmeinrichtung an dem Schraubenkopf lagefixiert werden. Zur Erhöhung der Winkelstabilität sind die korrespondierenden Anlageflächen 4,17 und 18,21 von Schraubenkopf und Adapter bzw. Adapter und Klemme paarweise oder jeweils mit nur einer Verzahnung versehen, wobei die korrespondierende Anlagefläche glatt oder rau ist. Es versteht sich, das auch andere Ausbildungen der Oberflächenbeschaffenheit zweckmäßig sein können.

Durch Verschwenkung des Schwenkelementes im entarretierten Zustand und Verdrehung der Klemme um die Längsachse des Schwenkelementes ist ein Verbindungsstab an der Schraube auf einfache Weise optimal positionierbar.

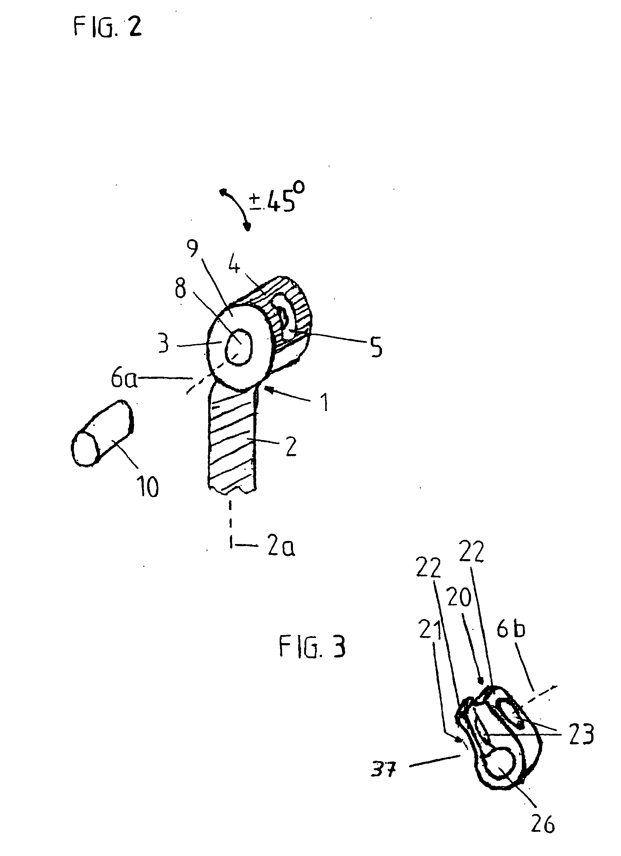

Die in Figur 2 gezeigte Ausführungsform entspricht im wesentlichen der nach Figur 1, wobei gleiche Bauteile mit gleichen Bezugsziffern versehen sind.

Im Unterschied zu Figur 1 ist der Schraubenkopf 3, der nach dem Ausführungsbeispiel stabförmig ausgeführt ist, mit seiner Hauptachse versetzt zu der Längsachse 2a des Schraubenschaftes angeordnet. Die Hauptachse entspricht hierbei der Längsachse des Schraubenkopfes und der Verschenkachse 6a des Adapters bzw. der Befestigungseinrichtung. Der seitliche Versatz entspricht hier ungefähr dem Radius des Schraubenschaftes.

Die Befestigungseinrichtung nach Figur 3 stellt eine Klemmeinrichtung in Form einer Bügelschelle dar, deren dem Schraubenkopf zugewandte Unterseite eine nicht-kugelkalottenförmige Einbuchtung aufweist, wenn die an die Kontur des Schraubenkopfes angepasst und flächig, insbesondere vollflächig an diesen anlegbar ist. Die die Einbuchtung 37 definierende Fläche entspricht einem Abschnitt einer Außenfläche eines nichtkugelförmigen Rotationskörpers, wie eines Zylinders oder Rotationsellipsoids. Die Einbuchtung ist vorzugsweise derart ausgeführt, dass sie vollflächig an einen Rotationskörper wie beispielsweise einen Zylinder oder ein Rotationsellipsoid anlegbar ist, wobei der Rotationskörper nicht kugelförmig ist und vorzugsweise eine Hauptachse aufweist, die länger als an die anderen Hauptachsen ist. Durch die nach dem Ausführungsbeispiel zylindrischkonkave Einbuchtung kann die Befestigungseinrichtung bei einer Verschwenkbewegung linienförmig oder flächig an dem Schraubenkopf oder einem Adapter anliegen und seitlich an diesem abgestützt werden, was bei einer Anlage an einem Kugelkopf bei kugelkalottenförmiger Einbuchtung nicht möglich wäre. Die Einbuchtung kann beispielsweise an einer ein- oder zweischenkeligen Befestigungseinrichtung für einen Verbindungsstab vorgesehen sein.

Anstelle der zylinderförmigen Umfangsflächen des Schraubenkopfes und des Adapters und/oder der Klemmeinrichtung können jeweils auch eine oder sämtliche der korrespondierenden Anlageflächen als Kugeloberflächenabschnitte ausgebildet sein, so dass das Schwenkelement eine Verschwenkbewegung in mehreren einen Winkel zueinander einschließenden Richtungen durchführen oder der Adapter und/oder die Klemmeinrichtung gegenüber dem Schraubenkopf in mehreren einen Winkel einschließenden Richtungen verschwenkbar sind.

Dadurch, dass bei der erfindungsgemäßen Vorrichtung bzw. bei der erfindungsgemäßen Knochenschraube das Schwenkelement bei der Schwenkbewegung eine Bewegung entlang der vorzugsweise zur Verschwenkebene hin geöffneten Ausnehmung des Schraubenkopfes durchführen und in der Ausnehmung eine bogenförmige Lageveränderung, vorzugsweise eines Kreisbogens, vollführen kann, ergeben sich somit besondere Vorteile des Erfindungsgegenstandes.

### Bezugszeichenliste

- 1: Knochenschraube
- 2: Schaft
- 2a: Längsachse
- 3: Kopf
- 4: Umfangsfläche
- 5: Ausnehmung
- 6: Schwenkelement
- 6a: Schwenkachse
- 6b: Richtung
- 7: Öse
- 8: Durchgangsbohrung
- 9: Stirnseite
- 10: Verschlussbolzen
- 14: Gewindeabschnitt
- 15: Adapter
- 16: Durchgangsbohrung
- 17,18: Fläche
- 20: Klemme
- 21: Unterseite
- 22: Schenkel
- 23: Durchgangsbohrung
- 25: Mutter
- 26: Aufnahme

## Patentansprüche

1. Knochenschraube (1) mit einem in einem Knochen befestigbaren Schraubenschaft (2) und einem Schraubenkopf (3), an dem eine Befestigungseinrichtung (20) mit einer Aufnahme (26) zur Halterung eines Verbindungsstabes befestigt ist, wobei die Befestigungseinrichtung in der Neigung gegen den Schraubenschaft (1) verschwenkbar ist, wobei der Schraubenkopf (3) eine Ausnehmung (5) aufweist, in der ein gegenüber dem Schraubenschaft verschwenkbares und lagefixierbares Schwenkelement (6) zur Befestigung der Befestigungseinrichtung (20) zur Festlegung des Verbindungsstabes und/oder eines Befestigungsbereichs des Verbindungsstabes und/oder eines gegebenenfalls vorgesehenen Adapters (15) angeordnet ist, wobei das Schwenkelement mit einem Befestigungsbereich (14) versehen ist, an dem der Adapter und/oder die Befestigungseinrichtung (20) zur Fixierung des Verbindungsstabes festgelegt ist und wobei ein Befestigungsmittel (25) vorgesehen ist, mittels dessen die Befestigungseinrichtung (20) betätigbar und gleichzeitig die Befestigungseinrichtung (20) oder der Adapter (15) unter Lagefixierung des Schwenkelementes gegen den Schraubenkopf (3) fixierbar ist, **dadurch gekennzeichnet, dass** ein separates Halteelement (10) vorgesehen ist, welches das Schwenkelement (6) im entarretierten Zustand gegen Entfernung aus der Ausnehmung (5) sichert und welches zugleich die Verschwenkachse des Schwenkelementes (6) definiert, und dass das Halteelement (10) in eine in der Ausnehmung (5) mündenden Bohrung (8) zumindest bis zu der Ausnehmung (5) eingeschoben und an einem in der Ausnehmung (5) angeordneten Endbereich des Schwenkelementes (6) festgelegt ist und einen in der Ausnehmung (5) angeordneten Vorsprung des Schwenkelementes (6) hintergreift oder in das Schwenkelement (6) eingreift.

2. Knochenschraube nach Anspruch 1, **dadurch gekennzeichnet, dass** die Ausnehmung (5) als Langloch ausgeführt ist.

3. Knochenschraube nach Anspruch 2, **dadurch gekennzeichnet, dass** sich das Langloch parallel zur Längsachse des Schraubenschaftes (2) erstreckt

4. Knochenschraube nach einem der Ansprüche 1 bis 3, **dadurch gekennzeichnet, dass** die Ausnehmung (5) an dem Schraubenkopf (3) auf Höhe des Schraubenschaftes (2) angeordnet ist.

5. Knochenschraube nach einem der Ansprüche 1 bis 4, **dadurch gekennzeichnet, dass** eine seitliche Führung für das Schwenkelement (6) vorgesehen ist, die im entarretierten Zustand des Schwenkelementes (6) mit diesem zusammenwirkt.

6. Knochenschraube nach einem der Ansprüche 1 bis 5, **dadurch gekennzeichnet, dass** der Schraubenkopf (3) zumindest im Anlagebereich zur Anlage des Adapters und/oder der Befestigungseinrichtung (20) zur Festlegung eines Verbindungsstabes die Form eines nichtrunden Rotationskörpers aufweist.

7. Knochenschraube nach einem der Ansprüche 1 bis 6, **dadurch gekennzeichnet, dass** der Schraubenkopf (3) exzentrisch zu dem Schraubenschaft (2) angeordnet ist.

8. Knochenschraube nach einem der Ansprüche 1 bis 7, **dadurch gekennzeichnet, dass** der Adapter zwischen dem Schraubenkopf der Knochenschraube und der Befestigungseinrichtung (20) zur Festlegung eines Verbindungsstabes und/oder dem Befestigungsbereich eines Verbindungsstabes angeordnet ist.

9. Knochenschraube nach Anspruch 8, **dadurch gekennzeichnet, dass** der Adapter (15) eine der Befestigungseinrichtung (20) zugeordnete Anlagefläche (18) aufweist, die plan, sphärisch konvex, sphärisch konkav oder konisch ausgebildet ist.

10. Knochenschraube nach Anspruch 8 oder 9, **dadurch gekennzeichnet, dass** der Adapter eine dem Schraubenkopf zugewandte Unterseite (19) mit einer bogenförmigen, nicht-kugelkalottenförmigen Einbuchtung aufweist, die der Außenfläche eines Rotationskörpers entspricht.

11. Knochenschraube nach einem der Ansprüche 1 bis 10, **dadurch gekennzeichnet, dass** die Befestigungseinrichtung zusätzlich gegenüber dem Schraubenschaft verdrehbar ist.

## Claims

1. Bone screw (1) with a screw shank (2) that can be fastened in a bone, and a screw head (3) on which a fastening device (20) with a receptacle (26) for mounting a connecting rod can be fixed, where the fastening device can be swiveled as regards its inclination relative to the screw shank (2), where the screw head (3) displays a recess (5), in which a swiveling element (6) that can at least be swiveled and fixed in position relative to the screw shank is located for fastening the fastening device (20) for fixing the connecting rod and/or a fastening area of the connecting rod and/or an optionally provided adapter (15), where the swiveling element (6) is provided with a fastening area (14) to which the adapter and/or the fastening device (20) for fixing the connecting rod is fixed, and where a fastening means (25) is provided, by means of which the fastening device (20) can be operated and, at the same time, the fastening device (20) or the adapter (15) can be fixed, thereby fixing the position of the swiveling element against the screw head (3), **characterized in that** a separate retaining element (10) is provided, which secures the swiveling element (6) against removal from the recess (5) when in unlocked state, and which furthermore, defines the pivoting axis of the swiveling element (6), and **in that** the retaining element (10) is inserted into a bore hole (8) ending in the recess (5), at least up to the recess (5), and is fixed on the end section of the swiveling element (6) located in the recess (5) and reaches behind a projection of the swiveling element (6) located in the recess (5) or engages the swiveling element (6) .

2. Bone screw according to Claim 1, **characterized in that** the recess (5) is designed as a slot.

3. Bone screw according to Claim 2, **characterized in that** the slot extends parallel to the longitudinal axis of the screw shank (2).

4. Bone screw according to one of Claims 1 to 3, **characterized in that** the recess (5) is located on the screw head (3) at the level of the screw shank (2).

5. Bone screw according to one of Claims 1 to 4, **characterized in that** a lateral guide for the swiveling element (6) is provided, which interacts with the swiveling element (6) in unlocked state of it.

6. Bone screw according to one of Claims 1 to 5, **characterized in that** the screw head (3) displays the form of a non-spherical body of revolution, at least in the contact area for positioning the adapter and/or the fastening device (20) for fixing a connecting rod.

7. Bone screw according to one of Claims 1 to 6, **characterized in that** the screw head (3) is located eccentrically relative to the screw shank (2).

8. Bone screw according to one of Claims 1 to 7, **characterized in that** the adapter is located between the screw head of the bone screw and the fastening device (20) for fixing a connecting rod and/or the fastening area of a connecting rod.

9. Bone screw according to Claim 8, **characterized in that** the adapter (15) displays a contact surface (18) assigned to the fastening device (20), which is of plane, spherically convex, spherically concave or conical design.

10. Bone screw according to Claim 8 or 9, **characterized in that** the adapter is provided with a lower surface (17) facing the screw head, the surface having an arc-shaped, non-spherical indentation corresponding to the outer surface of a revolution body.

11. Bone screw according to one of Claims 1 to 10, **characterized in that** the fastening device, additionally, is rotatable relatively to the screw shank.

## Revendications

1. Vis pour ostéosynthèse (1) avec une tige de vis (2) pouvant être fixée dans un os et une tête de vis (3) au niveau de laquelle un dispositif de fixation (20) avec un logement (26) pour maintenir une barre de raccordement est fixé, sachant que le dispositif de fixation peut être orienté en inclinaison contre la tige de vis (1), sachant que la tête de vis (3) présente un évidement (5) dans lequel un élément pivotant (6), pouvant être orienté par rapport à la tige de vis et dont la position peut être déterminée, est disposé pour fixer le dispositif de fixation (20) pour blocage de la barre de raccordement et/ou une zone de fixation de la barre de raccordement et/ou un adaptateur (15) prévu le cas échéant, sachant que l'élément pivotant est muni d'une zone de fixation (14) au niveau de laquelle l'adaptateur et/ou le dispositif de fixation (20) est bloqué pour fixation de la barre de raccordement et sachant qu'un moyen de fixation (25) est prévu pour permettre l'actionnement du dispositif de fixation (20) et en même temps, la fixation du dispositif de fixation (20) ou de l'adaptateur (15), la position de l'élément pivotant étant fixée, contre la tête de vis (3), **caractérisée en ce qu'**il est prévu un élément de retenue (10) séparé qui empêche l'élément pivotant (6), débloqué, de sortir de l'évidement (5) et qui définit, en même temps, l'axe de pivotement de l'élément pivotant (6) et **en ce que** l'élément de retenue (10) est introduit dans un trou (8) débouchant dans l'évidement (5) au moins jusqu'à l'évidement (5) et est bloqué au niveau d'une zone à l'extrémité, disposée dans l'évidement (5), de l'élément pivotant (6) et saisit par derrière une saillie, disposée dans l'évidement (5), de l'élément pivotant (6) ou est en prise dans l'élément pivotant (6).

2. Vis pour ostéosynthèse selon la revendication 1, **caractérisée en ce que** l'évidement (5) est réalisé en tant que trou longitudinal.

3. Vis pour ostéosynthèse selon la revendication 2, **caractérisée en ce que** le trou longitudinal s'étend parallèlement à l'axe longitudinal de la tige de vis (2).

4. Vis pour ostéosynthèse selon l'une quelconque des revendications 1 à 3, **caractérisée en ce que** l'évidement (5) est disposé au niveau de la tête de vis (3) à la hauteur de la tige de vis (2).

5. Vis pour ostéosynthèse selon l'une quelconque des revendications 1 à 4, **caractérisée en ce qu'**il est prévu, pour l'élément pivotant (6), un guidage latéral concourant avec l'élément pivotant (6), celui-ci étant débloqué.

6. Vis pour ostéosynthèse selon l'une quelconque des revendications 1 à 5, **caractérisée en ce que** la tête de vis (3) présente, au moins dans la zone de positionnement, pour placement de l'adaptateur et/ou du dispositif de fixation (20) pour blocage d'une barre de raccordement, la forme d'un corps rotatif non arrondi.

7. Vis pour ostéosynthèse selon l'une quelconque des revendications 1 à 6, **caractérisée en ce que** la tête de vis (3) est disposée excentrée par rapport à la tige de vis (2).

8. Vis pour ostéosynthèse selon l'une quelconque des revendications 1 à 7, **caractérisée en ce que** l'adaptateur est disposé entre la tête de vis de la vis pour ostéosynthèse et le dispositif de fixation (20) pour blocage d'une barre de raccordement et/ou la zone de fixation d'une barre de raccordement.

9. Vis pour ostéosynthèse selon la revendication 8, **caractérisée en ce que** l'adaptateur (15) présente une surface de positionnement (18) affectée au dispositif de fixation (20) et conçue plane, convexe sphériquement, concave sphériquement ou conique.

10. Vis pour ostéosynthèse selon la revendication 8 ou 9, **caractérisée en ce que** l'adaptateur présente un côté inférieur (19) orienté vers la tête de vis, avec un retrait en forme d'arc et de calotte non sphérique et correspondant à la face extérieure d'un corps rotatif.

11. Vis pour ostéosynthèse selon l'une quelconque des revendications 1 à 10, **caractérisée en ce que** le dispositif de fixation peut être en outre déplacé par rapport à la tige de vis.
